Europäisches Patentamt

European Patent Office

Office européen des brevets

(11) Numéro de publication: **0 234 974**
**B1**

(12) **FASCICULE DE BREVET EUROPEEN**

(45) Date de publication du fascicule du brevet:
**08.11.89**

(21) Numéro de dépôt: **87400090.4**

(22) Date de dépôt: **16.01.87**

(51) Int. Cl.⁴: **B 01 J 29/20** // C07C5/27, C10G45/64

(54) Catalyseur contenant une mordenite, sa préparation et son application à l'isomérisation de coupes riches en paraffines normales.

(30) Priorité: **22.01.86 FR 8600992**

(43) Date de publication de la demande:
**02.09.87 Bulletin 87/36**

(45) Mention de la délivrance du brevet:
**08.11.89 Bulletin 89/45**

(84) Etats contractants désignés:
**DE ES GB**

(56) Documents cités:
**DE-A- 2 342 481**
**FR-A- 2 203 676**
**US-A- 3 598 724**
**US-A- 3 796 766**

(73) Titulaire: **INSTITUT FRANCAIS DU PETROLE, 4, Avenue de Bois-Préau, F-92502 Rueil-Malmaison (FR)**

(72) Inventeur: **Dufresne, Pierre, 67, rue Georges Sand, F-92500 Rueil Malmaison (FR)**
Inventeur: **Franck, Jean-Pierre, 24, avenue Ivan Tourgueneff, F-78300 Bougival (FR)**
Inventeur: **Raatz, Francis, 17, rue Michelet, F-92500 Rueil Malmaison (FR)**
Inventeur: **Travers, Christine, 12, Bd du Général de Gaulle, F-92500 Rueil Malmaison (FR)**

## Description

La présente invention concerne un catalyseur comprenant une mordénite particulière, au moins un métal du groupe IVA de la classification périodique des éléments (Handbook of Chemistry and Physics 6ème édition 1980-81) et au moins un métal du groupe VIII de ladite classification, et éventuellement une matrice ou liant.

La présente invention concerne également les procédés de préparation de ce catalyseur et son utilisation notamment pour l'hydroisomérisation de coupes riches en paraffines normales ayant 4, 5, 6 ou 7 atomes de carbone par molécule.

L'isomérisation des paraffines normales de faible poids moléculaire est d'une importance considérable dans l'industrie pétrolière, vu l'indice d'octane particulièrement élevé des isoparaffines.

Il est intéressant de pouvoir transformer les n-paraffines $C_4$-$C_7$ et surtout $C_5$-$C_6$ en isoparaffines afin d'obtenir un carburant à haut indice d'octane. Ce procédé est intéressant pour améliorer les fractions d'essence légère et en particulier, les fractions de tête de distillation directe.

Il existe 3 types différents de procédés d'isomérisation:

— les procédés basse température (environ 20 à 130°C), utilisant un catalyseur de type Friedel et Crafts, tel que le chlorure d'aluminium,

— les procédés «moyenne» température (environ 150°C) utilisant comme catalyseur un métal supporté tel que le platine sur alumine halogénée,

— les procédés haute température (250°C et plus), mettant en oeuvre des supports zéolithiques associés à un métal hydrogénant du groupe VIII.

Quel que soit le type de catalyseurs utilisés, la réaction d'isomérisation est généralement accompagnée d'une réaction de craquage plus ou moins importante selon les catalyseurs et les conditions opératoires.

Les procédés «haute température» qui font l'objet de très nombreux brevets depuis une vingtaine d'années utilisent, pour la grande majorité d'entre eux, une zéolithe modifiée plus ou moins profondément et plus particulièrement une mordénite généralement sous forme acide, avec ou sans promoteurs d'hydrogénation.

On peut en particulier citer des brevets de la société SHELL utilisant des catalyseurs à base de mordénites modifiées selon des procédés particuliers: US-A-2 181 928, US-A-2 272 737, US-A-3 190 939, US-A-3 442 794, US-A-3 475 345, US-A-3 836 597, US-A-3 842 114, US-A-4 359 409 et US-A-4 400 576.

On peut également citer les brevets de la société ESSO utilisant des mordénites désaluminées partiellement US-A-3 480 539, US-A-3 506 400 et un brevet de la société MOBIL US-A-3 551 353.

Deux modes de dépôt de métal sont envisagés:

— dépôt du métal sur la zéolithe modifiée, c'est le cas de la plupart des brevets précités,

— dépôt du ou des métaux sur un liant inerte, alumine par exemple et mélange physique avec la zéolithe sous forme protonique (brevets de la société MOBIL US-A-3 432 568 et US-A-4 374 926 et brevet de la société UOP US-A-3 632 835).

Des traitements particuliers de la zéolithe, tels que l'introduction de fluor ont également été décrits dans les brevets US-A-3 932 554 et US-A-3 413 370.

La mordénite est caractérisée par un rapport atomique Si/Al compris généralement entre 4 et 6; sa structure cristalline est constituée d'enchaînements de tétraèdres de base $SiO_4$ et $AlO_4$, générateurs de deux types de canaux: des canaux à ouverture dodécagonale (contour à 12 oxygènes) et des canaux à ouverture octogonale (contour à 8 oxygènes).

Il existe cependant 2 types de mordénite, qui se distinguent par leur propriétés d'adsorption: la forme dite à larges pores, toujours synthétique, qui adsorbe les molécules telles que le benzène (diamètre cinétique = $6,6 \times 10^{-10}$ m) et la forme dite à petits pores, naturelle ou synthétique, qui n'adsorbe que des molécules de diamètre cinétique inférieur à $4,4 \times 10^{-10}$ m environ. Ces mordénites se distinguent également par des différences morphologiques — aiguilles pour la mordénite dite à petits pores, sphérulites pour la mordénite dite à larges pores — et structurales: présence ou non de défauts. Dans toute la littérature précitée, c'est la mordénite dite à larges pores qui est utilisée.

Le catalyseur objet de la présente invention, particulièrement sélectif pour l'hydroisomérisation de coupes riches en paraffines normales ayant de 4 à 7 atomes de carbone par molécule comprend une mordénite de structure particulière préparée à partir d'une mordénite dite à petits pores dans des conditions telles que la mordénite obtenue possède la capacité d'adsorber la molécule de benzène (diamètre cinétique: $6,6 \times 10^{-10}$ m) tout en ayant conservé en majeure partie, la morphologie de la mordénite de départ.

Ce procédé particulier de fabrication de la mordénite utilisée dans le catalyseur selon la présente invention a été décrit par la démanderesse dans la demande de brevet européen EP-A-196 965 publiée le 8 octobre 1986, et qui en consequence fait partie de l'état de la technique selon l'Art. 54(3) CBE.

La description de la préparation de la mordénite utilisée dans le catalyseur de la présente invention est incorporée ci-après par voie de référence.

De façon plus précise le catalyseur de la présente invention renferme en poids:

a) de 10 à 99,99%, de préférence de 35 à 85%, d'une manière avantageuse de 40 à 85% et, d'une manière la plus préférée de 60 à 85% d'une mordénite adsorbant des molécules de diamètre cinétique supérieur à $6,6 \times 10^{-10}$ mètre (m) ayant un rapport atomique Si/Al d'environ 5 à 50 et de préférence d'environ 5 à 30, une teneur en sodium inférieure à 0,2% en poids et de manière préférée inférieure à 0,1% en poids, par rapport au poids de la mordénite sèche, un volume de maille, V, de la maille élémentaire de 2,73 à 2,78 nanomètres cube ($nm^3$) et de manière préférée de 2,74 à 2,77 $nm^3$, une capacité d'adsorption de benzène supérieure à 5% et de préférence supérieure à 8% poids par rapport au poids de mordénite sèche, une morphologie particulière, à savoir qu'elle se présente, en majeure partie, sous forme d'aiguilles.

Les aiguilles ont généralement une longueur comprise entre $2 \times 10^{-6}$ et $20 \times 10^{-6}$ m, et plus particu-

lièrement entre $3 \times 10^{-6}$ et $10 \times 10^{-6}$ m, et de préférence une longueur moyenne de $5 \times 10^{-6}$ m. Les faces hexagonales de ces aiguilles ont généralement une longueur comprise entre $0,5 \times 10^{-6}$ m et $4 \times 10^{-6}$ m et plus particulièrement entre $0,5 \times 10^{-6}$ m et $3 \times 10^{-6}$ m et une «hauteur» compris entre $0,1 \times 10^{-6}$ m et $2 \times 10^{-6}$ m et plus particulièrement entre $0,2 \times 10^{-6}$ m et $1 \times 10^{-6}$ m; de préférence les faces hexagonales ont en majeure partie (c'est-à-dire au moins 50% des faces) une longueur d'environ $1 \times 10^{-6}$ m et une «hauteur» d'environ $0,3 \times 10^{-6}$ m;

b) de 0 à 89,99%, de préférence de 15 à 60%, d'une manière avantageuse de 15 à 55%, et d'une manière la plus préférée de 15 à 35%, d'une matrice ou liant choisi dans le groupe formé par l'alumine, la silice, la magnésie, la zircone, les argiles naturelles et des mélanges de ces corps tels que par exemple silice-alumine; la matrice sera de préférence de l'alumine ou un mélange contenant une proportion prépondérante d'alumine;

c) de 0,005 à 15%, de préférence de 0,05 à 10% d'au moins un métal du groupe VIII de la classification périodique des éléments, les métaux préférés étant le platine, la palladium et le nickel et la teneur en métal étant de préférence de 0,05 à 1%, d'une manière la plus préférée de 0,1 à 0,6% dans le cas du palladium ou du platine et étant de 0,1 à 10% et d'une manière la plus préférée de 0,2 à 5% dans le cas du nickel;

d) de 0,005 à 10% de préférence de 0,01 à 5% et d'une manière la plus préférée de 0,1 à 4% d'au moins un métal du groupe IVA de la classification périodique des éléments, étain, germanium ou plomb et de préférence l'étain.

La somme des pourcentages en poids de tous les éléments contenus dans le catalyseur étant égale dans chaque cas à 100%.

Le rapport atomique entre le ou les métaux du groupe IVA et le ou les métaux du groupe VIII (IVA/VIII) est généralement de 0,25:1 à 20:1 et de préférence de 0,4:1 à 10:1.

Au sens de la présente invention on entend par mordénite se présentant en majeure partie sous forme d'aiguilles, une mordénite dont au moins 50% et de préférence au moins 75% et d'une manière la plus préférée au moins 85% en poids possède cette morphologie particulière. La mordénite utilisée dans le catalyseur de la présente invention est fabriquée à partir d'une mordénite dite à petits pores, par exemple une mordénite du commerce, dont la teneur en sodium est comprise généralement entre 4 et 6,5% en poids par rapport au poids de mordénite sèche, dont le rapport atomique Si/Al est compris généralement entre 4,5 et 6,5 et le volume de maille généralement de 2,77 à 2,80 nm³. Cette mordénite n'adsorbe que des molécules de diamètre cinétique inférieur à environ $4,4 \times 10^{-10}$ m.

Les différentes caractéristiques des mordénites sont mesurées par les méthodes ci-après:

— les rapports atomiques Si/Al globaux sont déterminés par analyse fluorescence X, les teneurs en sodium par absorption atomique,

— le volume de la maille et la cristallinité sont déterminés par diffraction X, l'échantillon étant préparé de manière analogue au mode opératoire de la norme ASTM D3942 80 établie pour la faujasite,

— la capacité d'adsorption en benzène de la mordénite est déterminée par gravimétrie. L'échantillon est préalablement désorbé à 300°C sous $10^{-4}$ Torr ($133,32 \; 10^{-4}$ Pa). L'adsorption est ensuite menée à 30°C pendant 4 heures sous une pression P de 28 Torr (3733 Pa) de benzène, ce qui correspond à un rapport P/Ps égal à 0,25, Ps étant la pression de vapeur saturante à la température de l'expérience.

Les volumes adsorbés sont calculés à partir de la densité de l'adsorbant sous forme liquide à la température de l'adsorption: d = 0,868.

Il existe diverses méthodes d'obtention de mordénite ayant les caractéristiques et la morphologie particuliere en aiguilles telles que définies ci-dessus, à partir d'une mordénite dite à petits pores.

Selon une méthode préférée la mordénite dite à petits pores utilisée, est soumise aux différents traitements suivants: les cations sodium sont échangés par des cations ammonium, en plongeant la mordénite dans une solution d'un sel d'ammonium ionisable de moralité généralement supérieure à 0,5, à une température généralement comprise entre 20°C et 150°C. Cet échange peut être répété plusieurs fois. Le produit ainsi obtenu après ces échanges cationiques, peut être lavé puis soumis à un traitement thermique en présence de vapeur d'eau, qui peut être effectué par la technique du self steaming (calcination en atmosphère confinée). La température est comprise entre 300 et 800°C et de préférence 400 et 700, pendant un temps supérieur généralement à 10 minutes et de préférence supérieur à 20 minutes. L'atmosphère de calcination contient au moins 1% et de préférence au moins 5% de vapeur d'eau. Dans le cas du self steaming, l'atmosphère est constituée essentiellement d'eau et d'ammoniac. Le produit ainsi obtenu peut être soumis à un traitement acide visant à extraire de l'aluminium du solide. Ce traitement peut s'effectuer en plongeant le produit dans un acide minéral ou organique fort de normalité comprise entre 0,1 et 12 N, à une température comprise entre 20 et 150°C et de préférence entre 80 et 150°C, pendant un temps supérieur de préférence à 10 minutes.

Ce produit ayant subi le traitement acide peut être lavé, par exemple à l'acide, puis lavé à l'eau. Selon une autre méthode de préparation de la mordénite selon l'invention, il est possible également d'obtenir un bon catalyseur en utilisant un protocole différent. La mordénite dite à petits pores forme sodique peut être traitée directement une ou plusieurs fois par un acide minéral ou organique, de normalité comprise entre 0,1 et 12 N à une température comprise entre 20 et 150°C et de préférence 80 et 150°C. Dans ce cas, la quantité d'aluminium extraite par ce traitement acide doit être au moins de 20%, ce qui veut dire que le rapport Si/Al doit être d'au moins environ 6,5. Il est éventuellement possible de parfaire ensuite l'échange des cations sodium en traitant le produit dans des solutions d'un sel d'ammonium ionisable. L'introduction d'un métal (par exemple du groupe VIII) se déroule ensuite selon un des protocoles décrits ci-après.

D'autres méthodes de désalumination peuvent être envisagées telles que l'attaque par l'acide fluorhydrique, l'acide chlorhydrique en phase gazeuse, ou le traitement par un fluorosilicate ou toute autre méthode connue de l'homme du métier.

Le catalyseur de la présente invention renferme également une matrice ou liant, au moins un métal du groupe VIII et au moins un métal du groupe IVA.

Dans le cas ou le catalyseur de la présente invention est destiné à être utilisé dans les réactions d'hydroisomérisation de n-paraffines il est préférable que le ou les métaux du groupe IVA et le ou les métaux du groupe VIII se trouvent simultanément sur la matrice ou simultanément sur la mordénite.

Les métaux du groupe VIII présents dans le catalyseur final peuvent être introduits avant mélange de la mordénite avec la matrice ou après mélange de la mordénite avec la matrice. Il est possible d'introduire par exemple d'abord au moins un métal du groupe IVA sur la matrice ou sur la mordénite puis après mélange du produit obtenu avec la mordénite ou la matrice d'introduire au moins un métal du groupe VIII de manière à ce que ledit métal se dépose, en majeure partie, sur la partie du support (matrice ou mordénite) sur laquelle le métal du groupe IVA a été introduit.

De manière plus précise dans le cas ou le catalyseur comprend au moins un métal du groupe IVA et au moins un métal du groupe VIII introduits en majeure partie (c'est-à-dire au moins 50% et de préférence au moins 75% et d'une manière la plus préférée au moins 95% en poids) sur la matrice le procédé de préparation du catalyseur selon la présente invention comprend de préférence les étapes suivantes:

a) On introduit au moins un métal du groupe IVA sur la matrice.

b) On mélange intimement le produit résultant de l'étape (a) avec la mordénite ayant les caractéristiques et la morphologie particulière en aiguille telles que définies ci-dessus.

c) On introduit au moins un métal du groupe VIII soit avant l'étape (b) simultanément ou après l'étape (a), soit après l'étape (b) de manière à déposer, en majeure partie (c'est-à-dire au moins 50% et de préférence au moins 75% et d'une manière la plus préférée au moins 95% en poids), ledit métal sur ladite matrice.

Dans le cas où le catalyseur comprend au moins un métal du groupe IVA et au moins un métal du groupe VIII introduits, en majeure partie, sur la mordénite, le procédé de préparation du catalyseur selon la présente invention comprend de préférence les étapes suivantes:

a) On introduit au moins un métal du groupe IVA sur la mordénite ayant les caractéristiques et la morphologie particulière en aiguilles, telles que définies ci-dessus.

b) On mélange intimement le produit résultant de l'étape (a) avec la matrice.

c) On introduit au moins un métal du groupe VIII soit avant l'étape (b) simultanément ou après l'étape (a), soit après l'étape (b) de manière à déposer, en majeure partie (c'est-à-dire au moins 50% et de préférence au moins 75% et d'une manière la plus préférée au moins 95% en poids), ledit métal sur la mordénite.

Dans le cas ou les métaux sont introduits sur la matrice ou liant, le métal du groupe IVA peut être incorporé à cette matrice par toute méthode connue de l'homme du métier, conduisant à une répartition homogène du métal sur ladite matrice, par exemple par coprécipitation, cogélation ou imprégnation. Le métal du groupe VIII peut être introduit simultanément par exemple par coimprégnation, mais on préfère en général l'introduire séparément après avoir introduit le métal du groupe IVA; dans ce cas le métal du groupe IVA est introduit à l'aide d'un sel par comalaxage ou imprégnation à sec de la matrice, ou même par coprécipitation; le métal du groupe IVA peut également être introduit à l'aide de composés organométalliques dudit métal en milieu organique.

Dans le cas où la matrice est de l'alumine le sel soluble approprié du métal du groupe IVA peut être ajouté directement à l'hydrosol d'alumine, après gélification et traitements thermiques on obtient une combinaison intime dudit métal et de l'alumine. Il est également possible d'imprégner la matrice aluminique à l'aide d'un sel soluble décomposable du métal du groupe IVA le solvant utilisé est généralement l'eau. Les sels préférés des métaux du groupe IVA sont les chlorures, les nitrates, les sulfates, les acétates et les complexes amminés. Les autres composés également utilisables sont en particulier les tétraalkyls et les tétraalkoxy-métaux. D'une manière plus précise on préfère employer les composés suivants: pour l'étain le chlorure stanneux ou stannique, pour le germanium, le tétrachlorure de germanium pour le plomb le nitrate de plomb. Pour tous ces sels à la solution purement aqueuse, on préférera une solution fortement acide ayant un pH inférieur à environ 3 et de préférence inférieur à environ 1. L'agent d'acidification de la solution peut être un acide organique ou minéral. A titre d'exemples d'acides on peut citer l'acide chlorhydrique, l'acide nitrique, l'acide oxalique, l'acide malonique, l'acide citrique, l'acide malique, l'acide formique et l'acide tartrique.

Dans le cas ou l'imprégnation de la matrice est effectuée à l'aide d'un composé organométallique d'un métal du groupe IVA on peut citer à titre de composés préférés le tétrabutylétain, le tétraméthylétain, le diphénylétain, le tétrapropylgermanium et le tétraéthylplomb.

Après imprégnation du métal du groupe IVA, le métal du groupe VIII est par exemple introduit à l'aide d'une solution d'au moins un composé dudit métal du groupe VIII. Dans le cas où le métal du groupe VIII est le platine on utilisera de préférence une solution d'acide hexachloroplatinique, le platine se déposera alors sur la matrice par échange anionique. Il est également possible d'utiliser la technique dite de l'imprégnation à sec.

La matrice sur laquelle ont été déposés le ou les métaux du groupe IVA et le ou les métaux du groupe VIII est ensuite mélangée intimement à la mordénite ayant les caractéristiques et la morphologie particulières décrites ci-dessus, puis le mélange est mis en forme par tout moyen connu de l'homme du métier par exemple par extrusion, pastillage ou drageïfication. Le produit est ensuite séché et généralement calciné entre 300 et 600°C environ.

Une autre méthode préférée de préparation du catalyseur selon l'invention consiste à n'introduire le métal du groupe VIII qu'après avoir mélangé intimement la matrice imprégnée du métal du groupe IVA avec la mordénite. Dans ce cas l'introduction du métal du groupe VIII peut se faire soit avant, soit après la mise en forme. On utilisera un composé de métal du groupe VIII permettant audit métal de se déposer, en majeure partie, sur la matrice. On emploiera dans ce cas avantageusement une solution d'un composé minéral dudit métal. Dans le cas du platine on emploiera de préférence l'acide hexachloroplatinique.

Dans le cas où les métaux sont introduits sur la mordénite on préfère introduire d'abord le métal du groupe IVA par toute méthode connue de l'homme du métier et de préférence par imprégnation à l'aide d'une solution de l'un des sels des métaux, de préférence les sels cités supra en particulier les composés organométalliques desdits métaux, puis introduire le métal du groupe VIII. La mordénite contenant les métaux du groupe IVA et du groupe VIII est ensuite mélangée intimement à la matrice puis le mélange est mis en forme, séché et généralement calciné par exemple entre 300 et 600°C.

Une autre méthode préférée consiste à introduire seulement le métal du groupe IVA sur la mordénite puis à mélanger le produit résultant avec la matrice et à introduire ensuite le métal du groupe VIII, éventuellement après mise en forme à l'aide d'un composé dudit métal permettant de déposer ce métal, en majeure partie sur la mordénite. On emploiera dans ce cas avantageusement une solution d'un complexe organique dudit métal. Dans le cas du platine, on emploiera de préférence le platine tétrammine. Le platine dans ce cas se déposera sur la mordénite par échange cationique. Le produit résultant est si nécessaire mis en forme, puis séché et généralement calciné entre 300 et 600°C.

On peut également déposer en premier lieu le métal du groupe VIII sur la matrice, par exemple dans le cas du platine par échange anionique avec de l'acide hexachloroplatinique, ou sur la mordénite, par exemple par échange cationique avec un sel de platine tétrammine. Les deux produits ainsi obtenus peuvent être ensuite:

— soit directement imprégnés avec le métal du groupe IVA selon les méthodes décrites ci-dessus, puis mélangés à la mordénite et au liant respectivement, et, mis en forme par toute méthode connue de l'Homme du Métier,

— soit mélangés à la mordénite et au liant respectivement, et mis en forme. Le métal du groupe IVA sera alors déposé sur le produit mis en forme par toute méthode déjà décrite plus haut.

On pourra également, mélanger la matrice et la mordénite, les mettre en forme, puis déposer le métal du groupe VIII par échange anionique ou cationique. Le métal du groupe IVA par exemple l'étain sera alors déposé sur le produit ainsi obtenu par toute méthode déjà décrite supra.

Quelle que soit la méthode de dépôt du métal du groupe IVA utilisée, il est préférable que ce métal soit présent dans le catalyseur final dans un état d'oxydation supérieur à celui du métal élémentaire. Dans le cas de l'étain, ceci signifie que les meilleurs résultats sont obtenus quand pratiquement tout l'étain est à l'état d'oxydation + 2 ou + 4 et en particulier sous forme d'oxyde d'étain.

L'utilisation de catalyseurs selon l'invention, de préférence préparés selon l'une des méthodes décrites ci-dessus, lesdits catalyseurs contenant la proportion indiquée de chacun des éléments et contenant la mordénite de morphologie (aiguilles) et de caractéristiques particulières entraîne un gain d'activité et de sélectivité important dans la réaction d'hydroisomérisation de coupes riches en paraffines normales ayant de 4 à 7 atomes de carbone par molécule, par rapport aux catalyseurs d'hydroisomérisation de l'art antérieur.

Le catalyseur selon l'invention de préférence préparé selon l'une des méthodes décrites ci-dessus peut être utilisé pour isomériser les n-paraffines à 4, 5, 6 ou 7 atomes de carbone par molécule et en particulier les n-paraffines à 5 ou 6 atomes de carbone par molécule dans les conditions suivantes.

Suivant l'invention, la charge riche en paraffines légères à 5 ou 6 atomes de carbone et l'hydrogène sont mis en contact avec un catalyseur du type décrit ci-dessus dans les conditions de l'isomérisation. Ce contact peut s'effectuer en utilisant le catalyseur en lit fixe, en lit fluidisée ou en batch (c'est-à-dire en discontinu).

Le procédé est mis en oeuvre entre 200 et 300°C et de préférence entre 250°C et 280°C, à des pressions partielles d'$H_2$ comprises entre la pression atmosphérique et 70 bars (7 MPa) et de préférence entre 5 et 50 bars (0,5 et 5 MPa). La vitesse spatiale peut être comprise entre 0,5 et 10 litres d'hydrocarbures liquides par litre de catalyseur et par heure et de préférence entre 1 et 5. Le rapport molaire $H_2$/charge peut varier entre de larges limites et est compris normalement entre 0,5 et 10 et de préférence entre 1 et 5. L'isomérisation étant une réaction équilibrée, l'isomérisat contient encore une quantité importante de n-paraffines non converties. Ces paraffines peuvent être séparées des isomères par exemple par distillation ou par fractionnement sur tamis moléculaire et recyclées dans l'unité d'isomérisation.

Les exemples qui suivent définissent l'invention sans en limiter la portée.

Les performances sont exprimées en terme de conversion du n-alkane (du n-hexane ou du n-butane) et de sélectivité en isomérisation et de selectivité en craquage et définies comme suit:

$$\text{Conversion} = \frac{\text{Masse de n-alkane entrée} - \text{masse de n-alkane sortie} \times 100}{\text{Masse de n-alkane entrée}}$$

$$\text{Sélectivité Isomérisation} = \frac{\Sigma(\text{Masse des isomères}) \times 100}{\Sigma(\text{Masse des produits de la réaction})}$$

$$\text{Sélectivité craquage} \;=\; \frac{\Sigma(\text{Masse des produits de } C_1 \text{ à } C_5 \text{ (ou de } C_1 \text{ à } C_3) \times 100}{\Sigma(\text{Masse des produits de la réaction})}$$

**Exemple 1:** Préparation du catalyseur A.

L'alumine utilisée comme liant pour la mise en forme est un gel d'alumine disponible sur le marché de surface spécifique 250 m$^2$/g, de volume poreux 0,7 cm$^3$/g, se présentant sous forme de poudre.

0,8% d'étain sont ensuite déposés sur cette alumine ainsi prétraitée, à partir d'une solution de chlorure d'étain, acidifiée par une solution d'acide chlorhydrique décanormale. L'alumine ainsi imprégnée est ensuite séchée une nuit à l'étuve à 120°C, puis mélangée intimement à la mordénite prétraitée selon la technique précédemment décrite et rappelée ci-dessous.

La matière première est une mordénite dite à petits pores référence Alite 150 de la Société Chimique de la Grande Paroisse. Sa formule chimique à l'état anhydre est: Na AlO$_2$(SiO$_2$)$_{5,5}$, et sa capacité d'adsorption de benzène est de 1% en poids par rapport au poids de solide sec [volume de maille: 2,79 nm$^3$; teneur en sodium 5,3% (poids), diamètre cinétique des molécules adsorbées: 3,8 × 10$^{-10}$ m)]; 50 g de cette poudre sont plongés dans une solution 2 M de nitrate d'ammonium et la suspension est portée à 95°C pendant deux heures.

Le volume de la solution de nitrate d'ammonium engagée est égal à 4 fois le poids de mordénite sèche (V/P = 4). Cette opération d'échange cationique est recommencée 3 fois. Après le 3ème échange, le produit est lavé à l'eau, à 20°C pendant 20 minutes avec un rapport V/P égal à 4. La teneur en sodium, exprimée en pourcentage en poids par rapport au poids sec passe de 5,3 à 0,1%. Le produit est ensuite filtré et soumis à une calcination en atmosphère confinée (self steaming) à 600°C pendant 2 heures.

On procède ensuite à une attaque acide avec de l'acide chlorhydrique 0,58 N, en portant le produit à reflux dans la solution aqueuse d'acide chlorhydrique à 90°C pendant 2 heures avec un rapport V/P égal à 8. Le produit est ensuite filtré, lavé à l'acide chlorhydrique 0,1 N puis à l'eau.

Le rapport atomique Si/Al de cette mordénite est égal à 12, son volume de maille à 2,750 nm$^3$, son taux de sodium à 300 ppm, sa capacité d'adsorption de benzène à 9,6% poids par rapport au poids de solide sec et le diamètre cinétique des molécules adsorbées est de 6,8 10$^{-10}$ m. La morphologie de cette mordénite est en forme d'aiguilles de longueurs moyennes 5 × 10$^{-6}$ m dont les faces hexagonales one une longueur d'environ 1 × 10$^{-6}$ m et une hauteur d'environ 0,3 × 10$^{-6}$ m.

Après malaxage, le mélange constitué de 25% d'alumine imprégnée d'étain et de 75% de mordénite est forcé au travers d'une filière. Les extrudés de diamètre 1,2 × 10$^{-3}$ m sont ensuite séchés et calcinés. 0,4% de platine sont ensuite déposés sur ce support par échange anionique à partir d'une solution d'acide héxachloroplatinique. Les extrudés sont ensuite séchés à l'étuve à 120°C, et calcinés sous air sec à 500°C. Le catalyseur obtenu renferme en poids: alumine 24,7%, mordénite 74,7%, étain 0,20%, platine 0,4%.

Le catalyseur ainsi obtenu est chargé dans une unité catalytique en lit fixe et réduit sous hydrogène à 450°C. Il est ensuite testé avec une charge de normal hexane dans les conditions suivantes: température 270°C, pression 30 bars (3 MPa), poids de n-hexane par unité de poids de mordénite et par heure: 2, rapport molaire hydrogène sur normal hexane: 4. Les performances indiquées dans le tableau I sont prises après 30 h de mise en régime du catalyseur.

**Exemple 2:** Préparation du catalyseur B.

Le catalyseur B diffère du catalyseur A en ce que l'étain est déposé sur la mordénite, avec du tétraméthyl étain comme précurseur.

La mordénite est portée à reflux dans une solution d'heptane, contenant la quantité de tétraméthyl étain correspondant à 0,5% poids d'étain, rincée à l'heptane puis séchée. Elle est ensuite mélangée intimement à l'alumine peptisée. Après malaxage le mélange est forcé au travers d'une filière. Les extrudés obtenus sont séchés et calcinés.

0,4% de platine sont déposés sur le support ainsi mis en forme par échange cationique avec le chlorure de platine tétramine Pt(NH$_3$)$_4$Cl$_2$. Les performances catalytiques obtenues figurent dans le tableau I.

**Exemple 3:** Préparation du catalyseur C.

Le catalyseur C diffère du catalyseur A décrit dans l'exemple 1 en ce que ce n'est plus l'étain mais 0,8% de germanium à partir du tétrachlorure de germanium, qui sont déposés sur l'alumine. Les étapes consécutives sont rigoureusement semblables à celles décrites dans l'exemple 1. Les performances obtenues figurent dans le tableau I.

**Exemple 4:** Préparation du catalyseur D.

Le catalyseur D diffère du catalyseur A décrit dans l'exemple 1 en ce que ce n'est plus l'étain mais 0,8% de plomb à partir de nitrate de plomb qui sont déposés sur l'alumine. Les étapes consécutives sont rigoureusement semblables à celles décrites dans l'exemple 1. Les performances obtenues figurent dans le tableau I.

**Exemple 5:** Préparation du catalyseur E (comparaison).

La mordénite utilisée pour la préparation du catalyseur E est préparée à partir de la même mordénite de départ que celle employée dans l'exemple 1, ce produit est juste échangé 4 fois dans des solutions 2 M de nitrate d'ammonium, mais non soumis à des traitements acide ou thermique. Le rapport Si/Al est égal à 5,5, le taux de sodium à 300 ppm, le volume de maille à 2,778 nm$^3$ et la capacité d'adsorption de benzène à 1,2% poids. Le catalyseur est préparé comme dans l'exemple 1, il renferme en poids les mêmes quantités d'alumine, de mordénite, d'étain et de platine que le catalyseur obtenu dans l'exemple 1.

Les performances résumées dans le tableau I, montrent que du faut de la structure bouchée de la mordénite (elle n'adsorbe que 1,2% poids de benzène) l'activité en isomérisation est faible.

*Exemple 6:*

Le catalyseur A préparé suivant l'invention a également été testé sur du normal butane dans les conditions suivantes: température 350°C, pression 30 bars (3 MPa), poids de normal butane par unité de poids de mordénite et par heure: 1,2, rapport molaire hydrogène sur normal butane = 4. Les performances de ce catalyseur après 30 h de mise en régime du catalyseur sont les suivantes:

conversion du n-butane: 80 %
sélectivité en isomérisation: 99,5%
sélectivité en craquage: 0,5%

*Exemple 7:* Préparation du catalyseur F selon l'invention.

Après avoir malaxé puis mis en forme la matrice peptisée (alumine et la mordénite préalablement prétraitée dont les caractéristiques sont décrites dans l'exemple 1, on dépose 0,5% de platine à partir d'une solution de complexe de platine tétramine afin que ce dernier se dépose préférentiellement sur la mordénite. Après calcination et réduction de la phase métallique, 0,5% d'étain sont déposés en portant à reflux le catalyseur dans une solution de tétrabutyl étain dans l'heptane. Après séchage, le catalyseur est réduit in situ. Ses performances figurent dans le tableau I.

Les performances énoncées dans le tableau I des catalyseurs A à D et F selon l'invention sont nettement supérieures à celles obtenues avec le catalyseur de comparaison E.

*Exemple 8:* Préparation du catalyseur G (comparaison).

Le catalyseur G diffère du catalyseur B décrit dans l'exemple 2 en ce que la mordénite utilisée est une mordénite larges pores sous forme de poudre référencée Zeolon 100 Na de la Société NORTON.

50 g de cette poudre sont portés à reflux 2 heures à 950°C dans une solution de nitrate d'ammonium. Cet échange est recommencé 2 fois. Après le dernier échange le produit est lavé à l'eau pendant 20 mn à 20°C, filtré et calciné en atmosphère confinée (self steaming) à 600°C pendant 2 heures. Ce traitement thermique est suivi d'une attaque acide avec de l'acide chlorhydrique 0,58 N. Le solide est porté à reflux dans une solution aqueuse d'acide chlorhydrique à 90°C pendant 2 heures, puis lavé à l'eau.

Le rapport atomique Si/Al de la zéolithe obtenue est égal à 12, le volume de la maille à 2,752 nm³, et le taux de sodium à 250 ppm. Ce produit contrairement aux mordénites selon l'invention, n'a pas une morphologie en forme d'aiguilles.

La mordénite ainsi obtenue est portée à reflux dans une solution d'heptane, contenant la quantité de tétraméthyl étain correspondant à 0,5% poids d'étain, rincée à l'heptane puis séchée. Elle est ensuite mélangée intimement à l'alumine peptisée. Après malaxage le mélange est forcé au travers d'une filière. Les extrudés obtenus sont séchés et calcinés.

0,4% de platine sont déposés sur le support ainsi mis en forme par échange cationique avec le chlorure de platine tétramine Pt(NH₃)₄Cl₂. Les performances catalytiques obtenues figurent dans le tableau I.

TABLEAU I

| Exemple | Catalyseur | Conversion % | Sélectivité Isomérisation % | Sélectivité craquage % |
|---------|-----------|------|------|------|
| 1 | A | 80 | 99,5 | 0,5 |
| 2 | B | 80 | 99,8 | 0,2 |
| 3 | C | 80 | 99,3 | 0,7 |
| 4 | D | 80 | 99,3 | 0,7 |
| 5 | E | 10 | 97,0 | 3 |
| 7 | F | 80 | 99,7 | 0,3 |
| 8 | G | 57 | 97,5 | 2,5 |

**Revendications**

1. Catalyseur renfermant en poids:
a) de 10 à 99,99% d'une mordénite adsorbant des molécules de diamètre cinétique supérieur à environ $6,6 \times 10^{-10}$ m, ayant un rapport atomique Si/Al d'environ 5 à 50, une teneur en sodium inférieure à 0,2% en poids, par rapport à la totalité de la mordénite sèche, un volume de maille V de la maille élémentaire de 2,73 à 2,78 nanomètres cube, une capacité d'adsorption de benzène supérieure à 5% poids par rapport au poids de mordénite sèche, ladite mordénite, se présentant, en majeure partie, sous forme d'aiguilles,
b) de 0 à 89,99% d'une matrice choisie dans le groupe formé par l'alumine, la silice, la magnésie, la zircone, les argiles naturelles et des mélanges de ces corps,
c) de 0,005 à 15% d'au moins un métal du groupe VIII de la classification périodique des éléments et,
d) de 0,005 à 10% d'au moins un métal du groupe IVA de la classification périodique des éléments, choisi dans le groupe constitué par l'étain, le germanium et le plomb.

2. Catalyseur selon la revendication 1 dans lequel la mordénite est une mordénite adsorbant des molécules de diamètre cinétique supérieur à environ $6,6 \times 10^{-10}$ m, ayant un rapport atomique Si/Al d'environ 5 à 30, une teneur en sodium inférieure à 0,1% en poids, par rapport à la totalité de la mordénite sèche, un volume de maille V, de la maille élémentaire de 2,74 à 2,77 nanomètres cube, une capacité d'adsorption de benzène supérieure à 8% en poids de mordénite sèche, ladite mordénite se présentant, en majeure partie, sous forme d'aiguilles ayant une longueur comprise entre $2 \times 10^{-6}$ et $20 \times 10^{-10}$ m, dont les faces hexagonales ont une longueur comprise entre $0,5 \times 10^{-6}$ et $4 \times 10^{-6}$ m et une hauteur comprise entre $0,1 \times 10^{-6}$ et $2 \times 10^{-6}$ m.

3. Catalyseur selon la revendication 1 ou 2 caractérisé en ce qu'il renferme en poids:

a) de 35 à 85% de mordénite,

b) de 15 à 60% de matrice,

c) de 0,05 à 10% d'au moins un métal du groupe VIII,

d) de 0,01 à 5% d'au moins un métal du groupe IVA.

4. Catalyseur selon l'une des revendications 1 à 3 dans lequel le métal du groupe VIII est choisi dans le groupe formé par le platine, le palladium et le nickel, la teneur en platine ou palladium étant de 0,05 à 1% en poids et celle en nickel étant de 0,1 à 10% en poids.

5. Catalyseur selon l'une des revendications 1 à 4 dans lequel le métal du groupe IVA est l'étain.

6. Procédé de préparation d'un catalyseur selon l'une des revendications 3 à 5 dans lequel au moins un métal du groupe IVA et au moins un métal du groupe VIII sont introduit en majeure partie sur la mordénite comprenant les étapes suivantes:

a) on introduit au moins un métal du groupe IVA sur la mordénite,

b) on mélange le produit résultant de l'étape (a) avec la matrice,

c) on introduit au moins un métal du groupe VIII soit avant l'étape (b) simultanément ou après l'étape (a) soit après l'étape (b) à l'aide d'une solution d'un complexe organique dudit métal, de manière à déposer, en majeure partie, ledit métal sur la mordénite.

7. Procédé selon la revendication 6 comprenant les étapes suivantes:

a) on introduit au moins un métal du groupe IVA sur la mordénite,

b) on mélange le produit résultant de l'étape (a) avec la matrice,

c) on introduit au moins un métal du groupe VIII sur le produit résultant de l'étape (b) à l'aide d'une solution d'un complexe organique dudit métal de manière à déposer en majeure partie ledit métal sur la mordénite.

8. Procédé de préparation d'un catalyseur selon l'une des revendications 3 à 5, comprenant les étapes suivantes:

a) on introduit au moins un métal du groupe VIII sur la mordénite,

b) on mélange le produit résultant de l'étape (a) avec la matrice,

c) on introduit au moins un métal du groupe IVA soit avant l'étape (b) simultanément ou après l'étape (a), soit après l'étape (b) à l'aide d'une solution d'un complexe organique dudit métal, de manière à déposer, en majeure partie, ledit métal sur la mordénite.

9. Procédé de préparation d'un catalyseur selon l'une des revendications 3 à 5, comprenant les étapes suivantes:

a) on mélange la matrice avec la mordénite,

b) on introduit au moins un métal du groupe VIII sur le produit résultant de l'étape (a) à l'aide d'une solution d'un complexe organique dudit métal de manière à déposer, en majeure partie, ledit métal sur la mordénite,

c) on introduit au moins un métal du groupe IVA soit avant l'étape (b) sur le produit résultant de l'étape (a), soit simultanément à l'étape (b), soit après l'étape (b) à l'aide d'une solution d'un complexe organique dudit métal, de manière à déposer, en majeure partie, ledit métal sur la mordénite.

10. Utilisation du catalyseur selon l'une des revendications 1 à 5 ou du catalyseur préparé selon l'une des revendications 6 à 9 dans les réactions d'hydroisomérisation de coupes riches en n-paraffines possédant 4 à 7 atomes de carbone par molécule.

## Patentansprüche

1. Katalysator enthaltend (in Gew.-%):

a) 10 bis 99,99% eines Mordenits, welcher Moleküle von einem kinetischen Durchmesser von mehr als etwa $6,6 \times 10^{-10}$ m adsorbiert, mit einem Atomverhältnis Si/Al von etwa 5 bis 50, einem Natriumgehalt von weniger als 0,2 Gew.-%, bezogen auf das gesamte trockene Mordenit, ein Maschenvolumen V der Elementarmasche von 2,73 bis 2,78 Kubiknanometer, ein Benzoladsorptionsvermögen von mehr als 5 Gew.-%, bezogen auf das Gewicht trockenen Mordenits, wobei dieser Mordenit zum größeren Teil in Nadelform vorliegt,

b) 0 bis 89,99% einer Matrix, gewählt aus der Gruppe, die gebildet ist aus Aluminiumoxid, Siliziumoxid, Magnesiumoxid, Zirkonoxid, den natürlichen Tonen und den Gemischen dieser Körper,

c) 0,005 bis 15% wenigstens eines Metalls der Gruppe VIII des Periodensystems der Elemente und

d) 0,005 bis 10% wenigstens eines Metalls der Gruppe IVA des Periodensystems der Elemente (französische Schreibweise), gewählt aus der Gruppe, die aus Zinn, Germanium und Blei gebildet ist.

2. Katalysator nach Anspruch 1, bei dem der Mordenit ein Moleküle mit einem kinetischen Durchmesser von mehr als etwa $6,6 \times 10^{-10}$ m adsorbierender Mordenit ist, mit einem Atomverhältnis Si/Al von etwa 5 bis 30, einem Natriumgehalt von weniger als 0,1 Gew.-%, bezogen auf das gesamte trockene Mordenit, einem Maschenvolumen V der Elementarmasche von 2,74 bis 2,77 Kubiknanometer, eine Benzoladsorptionskapazität von mehr als 8 Gew.-% trockenen Mordenits, wobei dieses Mordenit zum größeren Teil in Form von Nadeln mit einer Länge zwischen $2 \times 10^{-6}$ und $20 \times 10^{-6}$ m vorliegt, deren Hexagonalflächen eine Länge zwischen $0,5 \times 10^{-6}$ und $4 \times 10^{-6}$ m und eine Höhe zwischen $0,1 \times 10^{-6}$ und $2 \times 10^{-6}$ m haben.

3. Katalysator nach Anspruch 1 oder 2, dadurch gekennzeichnet, daß er enthält (Gewicht):

a) 35 bis 85% Mordenit,

b) 16 bis 60% Matrix,

c) 0,05 bis 10% wenigstens eines Metalls der Gruppe VIII,

d) 0,01 bis 5% wenigstens eines Metalls der Gruppe IVA (französische Schreibweise des Periodensystems).

4. Katalysator nach einem der Ansprüche 1 bis 3, bei dem das Metall der Gruppe VIII gewählt ist aus der Gruppe, die gebildet wird durch Platin, Palladium und Nickel, wobei der Platin- oder Palladiumgehalt 0,05 bis 1 Gew.-%, und der des Nickels 0,1 bis 10 Gew.-% beträgt.

5. Katalysator nach einem der Ansprüche 1 bis 4, bei dem das Metall der Gruppe IVA (des Periodensystems in französischer Schreibweise) Zinn ist.

6. Verfahren zur Herstellung eines Katalysators nach einem der Ansprüche 3 bis 5, bei dem wenigstens ein Metall der Gruppe IVA und wenigstens ein Metall der Gruppe VIII zum größeren Teil auf den Mordenit gegeben werden, wobei das Verfahren die folgenden Stufen umfaßt:

a) man gibt wenigstens ein Metall der Gruppe IVA (des Periodensystems französischer Schreibweise) auf den Mordenit,

b) man mischt das aus der Stufe (a) resultierende Produkt mit der Matrix,

c) man führt wenigstens ein Metall der Gruppe VIII entweder vor der Stufe (b) gleichzeitig oder nach der Stufe (a) oder nach der Stufe (b) mit Hilfe einer Lösung eines organischen Komplexes dieses Metalls ein, derart, daß dieses Metall auf dem Mordenit zum größeren Teil abgeschieden wird.

7. Verfahren nach Anspruch 6, die folgenden Stufen umfassend:

a) man gibt wenigstens ein Metall der Gruppe IVA (des Periodensystems französischer Schreibweise) auf den Mordenit,

b) man mischt das aus der Stufe (a) resultierende Produkt mit der Matrix,

c) man gibt wenigstens ein Metall der Gruppe VIII auf das aus der Stufe (b) resultierende Produkt mit Hilfe einer Lösung eines organischen Komplexes dieses Metalls, derart, daß dieses Metall auf dem Mordenit zum größeren Teil abgeschieden wird.

8. Verfahren zum Herstellen eines Katalysators nach einem der Ansprüche 3 bis 5, die folgenden Stufen umfassend:

a) man gibt wenigstens ein Metall der Gruppe VIII auf den Mordenit,

b) man mischt das aus Stufe (a) resultierende Produkt mit der Matrix,

c) man gibt wenigstens ein Metall der Gruppe IVA (des Periodensystems französischer Schreibweise) entweder vor der Stufe (b) gleichzeitig oder nach der Stufe (a) oder nach der Stufe (b) mit Hilfe einer Lösung eines organischen Komplexes dieses Metalls, derart, daß zum größeren Teil dieses Metall auf dem Mordenit abgeschieden wird.

9. Verfahren zum Herstellen eines Katalysators nach einem der Ansprüche 3 bis 5, die folgenden Stufen umfassend:

a) man mischt die Matrix mit dem Mordenit,

b) man gibt wenigstens ein Metall der Gruppe VIII auf das aus der Stufe (a) stammende Produkt mit Hilfe einer Lösung eines organischen Komplexes dieses Metalls, derart, daß zum größeren Teil dieses Metall auf den Mordenit abgeschieden wird,

c) man führt wenigstens ein Metall der Gruppe IVA (des Periodensystems französischer Schreibweise) vor der Stufe (b) auf das aus der Stufe (a) stammende Produkt entweder gleichzeitig mit der Stufe (b) oder nach der Stufe (b) mit Hilfe einer Lösung eines organischen Komplexes dieses Metalls, derart, daß dieses Metall auf den Mordenit zum größeren Teil abgeschieden wird.

10. Verwendung des Katalysators nach einem der Ansprüche 1 bis 5 oder des nach einem der Ansprüche 6 bis 9 hergestellten Katalysators bei Hydroisomerisierungsreaktionen von Schnitten, die an n-Paraffinen, welche 4 bis 7 Kohlenstoffatome pro Molekül besitzen, reich sind.

## Claims

1. A catalyst containing by weight:

a) from 10 to 99.99% of a mordenite adsorbing molecules of a kinetic diameter larger than about $6.6 \times 10^{-10}$ m, having a Si/Al atomic ratio from about 5 to 50, a sodium content by weight lower than 0.2% of the total weight of dry mordenite, a volume V of elementary mesh from 2.73 to 2.78 cubic nanometers, a benzene adsorption capacity of more than 5% by weight with respect to the dry mordenite weight, said mordenite being in major part shaped as needles,

b) from 0 to 89.99% of a matrix selected from the group consisting of alumina, silica, magnesia, zirconia, natural clays or mixtures thereof,

c) from 0.005 to 15% of at least one metal from group VIII of the periodic classification of elements, and

d) from 0.005 to 10% of at least one metal group IVA of the periodic classification, selected from the group consisting of tin, germanium and lead.

2. A catalyst according to claim 1, wherein the mordenite has the capacity to adsorb molecules of a kinetic diameter larger than about $6.6 \times 10^{-10}$ m, has a Si/Al atomic ratio from about 5 to 30, a sodium content by weight lower than 0.1% by weight of the total dry mordenite weight, a volume of the elementary mesh from 2.74 to 2.77 cubic nanometers, a benzene adsorption capacity higher than 8% of the dry mordenite weight, said mordenite being in major part shaped as needles of 2 to 20 μm length having hexagonal faces of 0.5 to 4 μm length and of 0.1 to 2 μm height.

3. A catalyst according to claim 1 or 2, characterized by a content by weight of:

a) 35-85% of mordenite,
b) 15-60% of matrix,
c) 0.05-10% of at least one group VIII metal,
d) 0.01-5% of at least one group IVA metal.

4. A catalyst according to one of claims 1 to 3, wherein the group VIII metal is selected from the group consisting of platinum, palladium and nickel, the platinum or palladium content being from 0.05 to 1% by weight and the nickel content from 0.1 to 10% by weight.

5. A catalyst according to one of claims 1 to 4, wherein the group IVA metal is tin.

6. A process for preparing a catalyst according to one of claims 3 to 5, wherein at least one group IVA metal and at least one group VIII metal are introduced, in major part, on the mordenite, comprising the stepf of:

a) introducing at least one group IVA metal on mordenite,

b) admixing the product obtained in step (a) with the matrix,

c) introducing at least one group VIII metal either before step (b), during or after step (a), or after step (b), by means of a solution of an organic complex of

said metal, so as to deposit, in major part, said metal on mordenite.

7. A process according to claim 6, comprising the following steps of:

a) introducing at least one group IVA metal on mordenite,

b) admixing the resultant product from step (a) with the matrix,

c) introducing at least one group VIII metal on the product obtained from step (b) by means of a solution of an organic complex of said metal, so as to deposit, in major part, said metal on mordenite.

8. A process for preparing a catalyst according to any of claims 3 to 5, comprising the following steps of:

a) introducing at least one group VIII metal on mordenite,

b) admixing the resultant product from step (a) with the matrix,

c) introducing at least one group IVA metal either before step (b), during or after step (a), or after step (b), by means of a solution of an organic complex of said metal, so as to deposit, in major part, said metal on mordenite.

9. A process for preparing a catalyst according to any of claims 3 to 5, comprising the following steps of:

a) admixing the matrix with mordenite,

b) introducing at least one group VIII metal on the product obtained in step (a), by means of a solution of an organic complex of said metal, so as to deposit, in major part, said metal on mordenite,

c) introducing at least one group IVA metal on the product resulting from step (a) either before step (b) or simultaneously with step (b), or after step (b), by means of a solution of an organic complex of said metal, so as to deposit, in major part, said metal on mordenite.

10. The use of a catalyst according to any of claims 1 to 5, or of a catalyst prepared according to one of claims 6 to 9, in hydroisomerization reactions of cuts containing a high proportion of n-paraffins having 4 to 7 carbon atoms per molecule.